(19)

**Europäisches Patentamt**
**European Patent Office**
**Office européen des brevets**

(11) **EP 3 415 085 B1**

(12) **EUROPEAN PATENT SPECIFICATION**

(45) Date of publication and mention
of the grant of the patent:
**20.03.2024 Bulletin 2024/12**

(21) Application number: **17176000.2**

(22) Date of filing: **14.06.2017**

(51) International Patent Classification (IPC):
**G16H 40/63** (2018.01)    **A61B 5/24** (2021.01)
**A61B 5/00** (2006.01)

(52) Cooperative Patent Classification (CPC):
**G16H 40/63; A61B 5/0002; A61B 5/24**

(54) **A METHOD FOR PROCESSING SENSOR DATA IN A NEUROPROBE**

VERFAHREN ZUR VERARBEITUNG VON SENSORDATEN IN EINER NERVENSONDE

PROCÉDÉ DE TRAITEMENT DE DONNÉES DE CAPTEUR DANS UN NEUROPROBE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.12.2018 Bulletin 2018/51**

(73) Proprietors:
• **IMEC vzw**
**3001 Leuven (BE)**
• **Katholieke Universiteit Leuven, KU Leuven R&D**
**3000 Leuven (BE)**
• **Norges Teknisk-Naturvitenskapelige Universitet**
**7491 Trondheim (NO)**
• **VIB VZW**
**9052 Gent (BE)**

(72) Inventors:
• **YASSIN, Yahya Hussain**
**3001 Leuven (BE)**
• **CATTHOOR, Francky**
**3001 Leuven (BE)**
• **KJELDSBERG, Per Gunnar**
**3001 Leuven (BE)**
• **KLOOSTERMAN, Fabian**
**3001 Leuven (BE)**
• **SUN, Jyh-Jang**
**3001 Leuven (BE)**
• **COUTO, Joao**
**3001 Leuven (BE)**

(74) Representative: **AWA Sweden AB**
**Box 5117**
**200 71 Malmö (SE)**

(56) References cited:
**WO-A2-2008/057365      US-A1- 2005 090 756
US-A1- 2006 116 738**

• **BENOIT GOSSELIN ET AL: "A low-power
integrated neural interface with digital spike
detection and extraction", ANALOG
INTEGRATED CIRCUITS AND SIGNAL
PROCESSING, KLUWER ACADEMIC
PUBLISHERS, BO, vol. 64, no. 1, 15 August 2009
(2009-08-15), pages 3-11, XP019812292, ISSN:
1573-1979**
• **GORDILLO CAMILO ET AL: "Automatic channel
selection in neural microprobes: A combinatorial
multi-armed bandit approach", 2016 IEEE/RSJ
INTERNATIONAL CONFERENCE ON
INTELLIGENT ROBOTS AND SYSTEMS (IROS),
IEEE, 9 October 2016 (2016-10-09), pages
1844-1850, XP033011643, DOI:
10.1109/IROS.2016.7759293 [retrieved on
2016-11-28]**
• **Mohsen Judy ET AL: "Data Reduction
Techniques in Neural Recording Microsystems"
In: "Advances in Bioengineering", 8 July 2015
(2015-07-08), InTech, XP055276824, ISBN:
978-953-51-2141-1 DOI: 10.5772/59662, * page 297
***
• **MICHAEL RIZK ET AL: "A single-chip signal
processing and telemetry engine for an
implantable 96-channel neural data acquisition
system; A single-chip neural signal processing
and telemetry engine", JOURNAL OF NEURAL
ENGINEERING, INSTITUTE OF PHYSICS
PUBLISHING, BRISTOL, GB, vol. 4, no. 3, 20 July
2007 (2007-07-20), pages 309-321, XP020123746,
ISSN: 1741-2552, DOI: 10.1088/1741-2560/4/3/016**

- **RADUCANU BOGDAN C ET AL: "Time multiplexed active neural probe with 678 parallel recording sites", 2016 46TH EUROPEAN SOLID-STATE DEVICE RESEARCH CONFERENCE (ESSDERC), IEEE, 12 September 2016 (2016-09-12), pages 385-388, XP032980774, DOI: 10.1109/ESSDERC.2016.7599667 [retrieved on 2016-10-18]**
- **YAHYA H. YASSIN ET AL: "Algorithm/Architecture Co-optimisation Technique for Automatic Data Reduction of Wireless Read-Out in High-Density Electrode Arrays", ACM TRANSACTIONS ON EMBEDDED COMPUTING SYSTEMS., vol. 17, no. 3, 2 June 2018 (2018-06-02), pages 1-19, XP055750039, US ISSN: 1539-9087, DOI: 10.1145/3190854**

## Description

Technical Field

The invention generally relates to the field of neuroscience. More particularly, it is related to methods for processing sensor data in neuroprobes as well as neuroprobes with built-in sensor data processing functionality.

Background of the invention

In neuroscience, a large amount of research is based on the study of the activity of neurons in brains in vivo. The neuron activity are normally recorded extracellularly with electrodes implanted in brains, and raw data is transmitted through wires from the electrodes to a computer for processing.

Future electrode densities in neuroprobes are most likely to reach the limits of how much data we can transfer within the energy budget available on the neuroprobes, even while transmitting the data through electrical wiring. The reason is the fast growing amount of concurrent sensor nodes on a neuroprobe. Several recent research projects are aiming towards 10 000 electrodes on a single neuroprobe head. On top of that, for deep brain electrodes, the aim for future research is moving towards battery powered wireless solutions. This will reduce the amount of data that can be transmitted in raw form to the output even more. Hence, a need is present for data reduction in order to meet the stringent requirements for data transfer. Existing solutions are either taking too much area and energy overhead or they are implemented using non-scalable analog front-end circuitry. In the article titled "A low-power integrated neural interface with digital spike detection and extraction" by Benoit Gosselin et al from ANALOG INTEGRATED CIRCUITS AND ISGNAL PROCESSING, KLUWER ACADEMIC PUBLISHERS, BO, vol 64, no. 1, 15 August 2009 (2009-08-15), pages 3-11, XPO19812292, ISSN: 1573-1979, it is described a low-power integrated neural interface with digital spike detection and extraction.

In the article "Automatic channel selection in neural microprobes: A combinatorial multi-armed bandit approach" by GORDILLO CAMILO ET AL from 2016 IEEE/RSJ INTERNATIONAL CONFERENCE ON INTELLIGENT ROBOTS AND SYSTEMS (IROS), IEEE, 9 October 2016 (2016-10-09), pages 1844-1850, XP033011643,DOI: 10.1109/ IROS.2016.7759293 it is described a neural microprobe with automatic channel selection.

Summary

Accordingly, the present invention preferably seeks to mitigate, alleviate or eliminate one or more of the above-identified deficiencies in the art and disadvantages singly or in any combination.

According to a first aspect it is provided a method for processing a sample set of sensor data in a neuroprobe comprising a plurality of channels, wherein the sample set of sensor data comprises channel sensor data from each of the plurality of channels for a given time period, wherein the plurality of channels comprises target channels, each comprising target data comprising pre-selected features, and non-target channels, each being without target data, wherein the target channels and the non-target channels are sample specific, said method comprising identifying the target channels and the non-target channels by for each of the plurality of channels, capturing the channel sensor data, determining if the channel sensor data comprises the target data by comparing the channel sensor data with prior channel sensor data, and assigning as target channel if the channel sensor data comprises the target data, identifying selected channels by for each of the plurality of channels selecting as selected channel if assigned as target channel, forming and transmitting an aggregated sensor data package by for each of the selected channels forming a data package comprising channel sensor data, combining data packages from the selected channels into the aggregated sensor data package, and transmitting the aggregated sensor data package from the neuroprobe to a data processing device configured for analyzing channel sensor data.

The step of identifying selected channels further comprises for each of the non-target channels selecting as selected channels if placed within a spatial threshold from at least one of the target data channels.

The channels of the neuroprobe may comprise electrodes.

The target data may comprise a spike.

The spike may be determined by comparing the channel sensor data with a running threshold being a combination of a channel sensor data mean value and a channel sensor data standard deviation value.

The step of identifying the target channels and the non-target channels may be made in parallel for the plurality of channels, the step of identifying selected channels may be made in sequence for the plurality of channels, and the step of forming and transmitting an aggregated sensor data package may be made in parallel for the plurality of channels.

The step of selecting as selected channel if assigned as target channel may comprise setting a channel associated bit in a data memory.

The plurality of channels of the neuroprobe may be at least 1000 channels.

The plurality of channels may each comprise a unique identifier, and the plurality of channels may be divided into sub-groups based on the unique identifiers, wherein the step of identifying the target channels and the non-target channels further may comprise identifying a sub-group, and assigning as target channel if another channel within the sub-group is assigned as target channel.

According to a second aspect it is provided a neuroprobe comprising a plurality of channels, wherein the plurality of channels comprises target channels, each comprising target data comprising pre-selected features, and non-target channels, each being without target data, wherein the target channels and the non-target channels are sample specific, a target channels identifier for identifying the target channels and the non-target channels by for each of the plurality of channels, capturing a sample set of sensor data, wherein the sample set comprises channel sensor data from each of the plurality of channels for a given time period, determining if the channel sensor data comprises the target data by comparing the channel sensor data with prior channel sensor data, determining if the channel sensor data comprises the target data, and assigning as target channel if the channel sensor data comprises the target data, a selected channels identifier for identifying selected channels by for each of the plurality of channels selecting as selected channel if assigned as target channel, and a data transmitter for forming and transmitting an aggregated sensor data package by for each of the selected channels forming a data package comprising channel sensor data, combining data packages from the selected channels into the aggregated sensor data package, and transmitting the aggregated sensor data package from the neuroprobe to a data processing device configured for analyzing channel sensor data.

The selected channels identifier is further configured to for each of the non-target channels selecting as selected channels if placed within a spatial threshold from at least one of the target data channels.

The channels of the neuroprobe may comprise electrodes.

The target data may comprise a spike.

The spike may be determined by comparing the channel sensor data with a running threshold being a combination of a channel sensor data mean value and a channel sensor data standard deviation value.

The target channels identifier may be handling the plurality of channels in parallel, the selected channels identifier may be handling the plurality of channels in sequence, and the data transmitter may be handling the plurality of channels in parallel.

The step of selecting as selected channel if assigned as target channel may comprise setting a channel associated bit in a data memory.

The plurality of channels of the neuroprobe may be at least 1000 channels.

The plurality of channels may each comprise a unique identifier, and the plurality of channels may be divided into sub-groups based on the unique identifiers, wherein the step of identifying the target channels and the non-target channels may further comprise identifying a sub-group, and assigning as target channel if another channel within the sub-group is assigned as target channel.

According to a third aspect it is provided a system for analyzing neural activity, said system comprising a neuroprobe according to the second aspect, and an external data processing device configured for analyzing channel sensor data.

The external data processing device may be configured for analyzing the channel sensor data by extracting features from the channel sensor data and clustering the features.

## Brief description of the drawings

The above, as well as additional objects, features and advantages of the present invention, will be better understood through the following illustrative and non-limiting detailed description, with reference to the appended drawings, wherein:

Fig 1 illustrates general steps for processing sensor data captured by a neuroprobe;
Fig 2 illustrates three phases for pre-processing sensor data in the neuroprobe;
Fig 3 is a flow chart illustrating steps of a method for processing sensor data in the neuroprobe; and
Fig 4 is a flow chart illustrating a system for analyzing neural activity.

## Detailed description

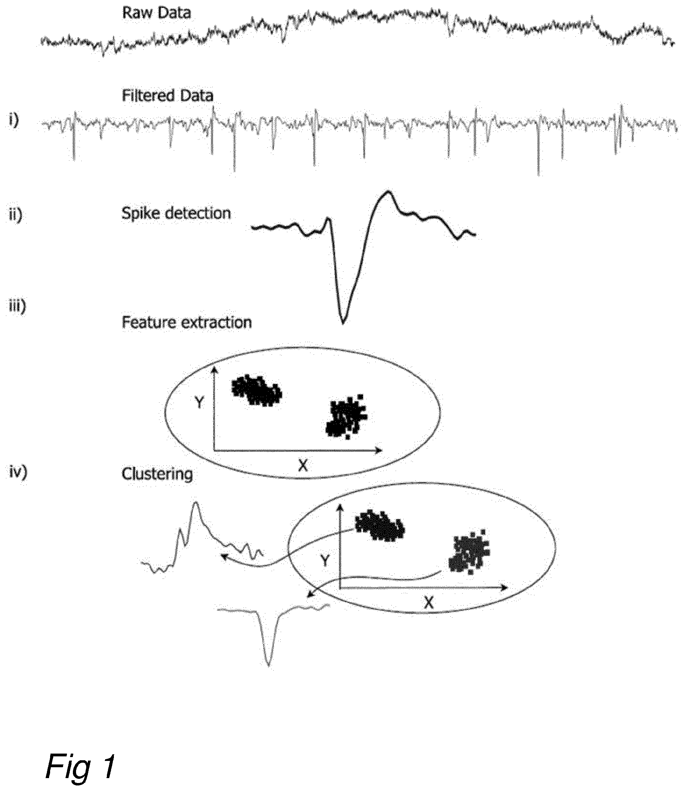

Within the field of neuroscience there are different approaches for processing data captured by a neuroprobe. In order to efficiently transform raw data captured by electrodes of the neuroprobe to data that can form basis for a decision, either for a physician or for a software-implemented decision algorithm, an approach illustrated in fig 1 may be used.

In a first step i), raw data formed by the electrodes is processed in that the raw data is filtered such that filtered data is formed. By filtering the raw data noise may be removed such that later steps can be made more efficiently and in a more reliable manner. Depending on how the filtered data is to be used different filter software and/or filter hardware may be used.

Based on the filtered data, in a second step ii) spike detection can be performed. A spike, or action potential, in the filtered data is reflecting that a potential of a neuron membrane has quickly risen and thereafter quickly dropped. By using signal processing algoritms such spikes can be detected in the filtered data.

When having detected a number of spikes these can be analyzed by using so-called feature extraction analysis as illustrated in step iii). The features may relate to a wave shape, an amplitude, a duration etc. In short and by way of example, different features can be extracted for the spikes using principal component analysis (PCA). After the feature extraction, the different spikes can be categorized on the basis of their features and clustered in different groups as illustrated in step iv), where each group represents a group of spikes sharing the same or very similar features.

Each of the data processing steps above results in a reduction of the amount of data. In order to reduce the amount of data that is transmitted from the neuroprobe to an external data processing device one or several of the steps may be made in the neuroprobe itself. An advantage of this is that less data needs to be transmitted from the neuroprobe to the external data processing device, which in turn implies that an increased number of electrodes can be used. In case wireless transmission is used, this will result in that the wireless transmit energy needed is reduced, which in turn can result in that high density neuroprobes are made possible also for wireless transmission based systems.

By performing the spike data detection in the neuroprobe, target data, i.e. sensor data comprising spike data or other data considered relevant for further analysis, can be identified and transmitted to the external data processing device. The sensor data not comprising target data, also referred to as non-target data, may also be transmitted if e.g. the non-target data is collected spatially close to a location where target data is collected. Thus, by having such approach an increased number of electrodes are made possible and/or wireless transmission of data from the electrodes to the external data processing device is made possible.

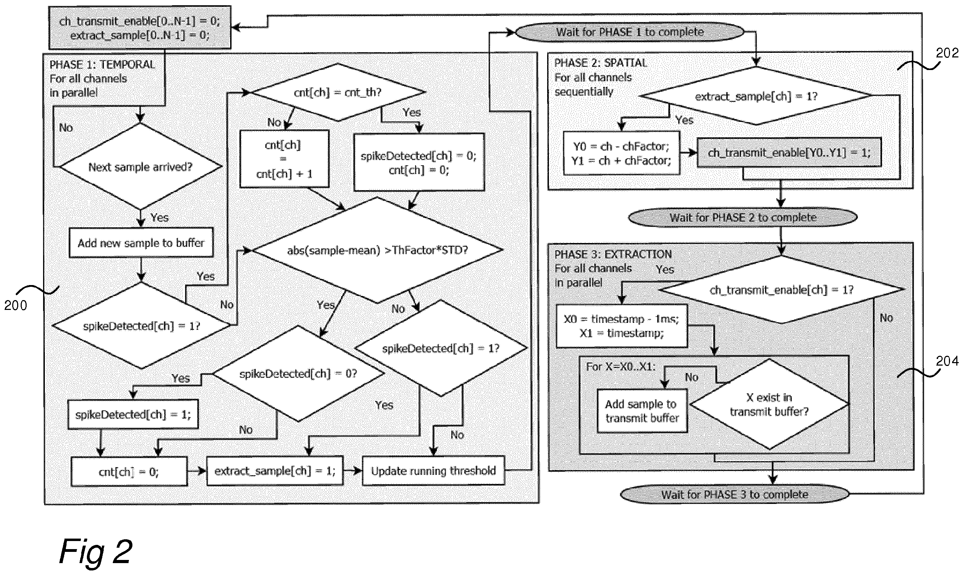

Fig 2 generally illustrates by way of example three phases for spike detection and data transmission that can be made by a processor in the neuroprobe provided with a plurality of channels. In a first phase 200, channels, such as electrodes, comprising target data, such as spike data, are identified. After having identified target channels, i.e. channels comprising target data, and non-target channels, i.e. channels not comprising target data, the plurality of channels are in a second phase 202 iterated and the target channels are marked as selected channels, i.e. channels comprising data relevant to transmit, and optionally also non-target channels spatially close to target channels. Finally, in a third phase 204 the target data from the selected channels are transmitted from the neuroprobe. All phases are performed for each sample per channel.

More in detail and by way of example, the first phase 200 may comprise a spike detection. Generally, this can be made by distinguishing between relevant data, herein referred to as spikes, caused by neural activity and other non-relevant data, such as noise, not caused by neural activity. This can be made by using a crude thresholding mechanism. As an example, the threshold mechanism may calculate the running mean and running standard deviation (STD) and, based on these a sample may be assessed based on the following formula:

$$|(sample - mean)| > ThFactor * STD$$

where ThFactor is a configurable factor used to tune the sensitivity of the threshold.

If the absolute value of a difference between the sample and the running mean is greater than ThFactor*STD, then a spikeDetected flag for the corresponding electrode (expressed as channel from now on) is marked (set to 1) if it is not set from before. An internal counter for the corresponding channel (cnt[ch]) is then reset to zero, before an extract_sample flag for the same channel is marked (set to 1). If the spikeDetected flag was marked from before, then the channel counter (cnt) is compared to a count threshold (cnt_th, set to be equal to the sampling frequency divided by 1000). If the counter has reached the count threshold, then it resets the counter (cnt[ch]) and the spikeDetected[ch] flag, before continuing to update the running threshold. If the absolute value of a sample crosses the threshold in the succeeding step, then the spikeDetected[ch] flag is marked (set to 1), and the counter (cnt[ch]) is reset to zero. The spikeDetected flag in combination with the cnt counter consist of the mechanism that marks the succeeding 1 ms samples once a threshold crossing is detected. A buffer can be used to extract the proceeding 1 ms of samples in phase 3.

The second phase 202 is a sequential phase that iterates through all channels one by one. If the extraction bit is marked on a channel, then it marks a ch_transmit_enable bit for the channel, and for a specific number (chFactor) of preceding and succeeding channels. This phase performs a spatial selection for data to be transmitted.

The third phase 204 is a parallel phase where it is checked for all electrodes simultaneously if their corresponding ch_transmit_enable bit is marked. If it is marked, then the electrodes (channels) transmits its sample. Otherwise it discards its sample. After phase 3 completes, the transmit enable bits are reset for all channels.

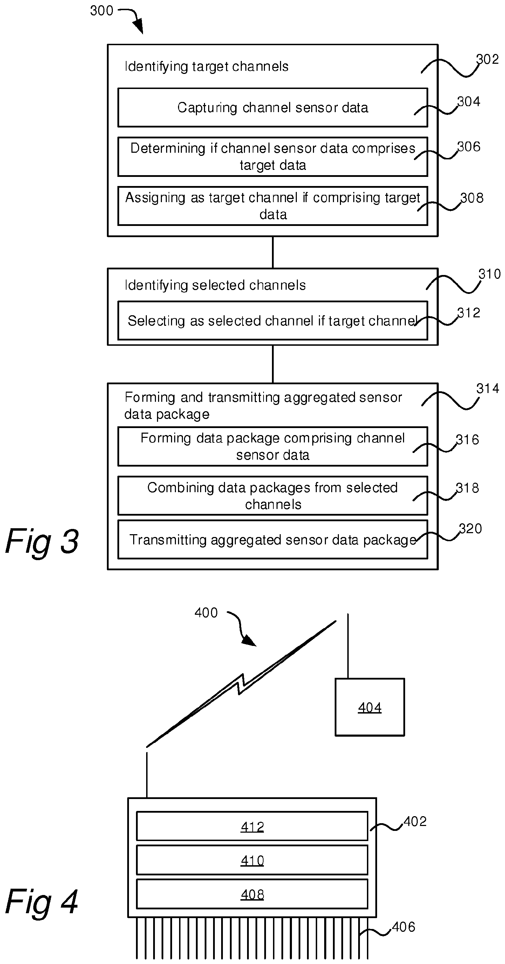

Based on the non-limiting example illustrated in fig 2 and described above, in fig 3 a general method 300 for processing a sample set of sensor data in a neuroprobe comprising a plurality of channels is illustrated by way of a flow chart. The sample set of sensor data comprises channel sensor data from each of the plurality of channels for a given time period.

In a first main step 302 target channels are identified in that channel sensor data for the plurality of channels are captured in a first sub-step 304, in that it is determined if the channel sensor data comprises target data comprising pre-selected features, e.g. features identifying a spike, in a second sub-step 306, and in that channels determined to comprise target data are assigned as target channels in a third sub-set 308. This first main step 302 may be performed in parallel for the plurality of channels.

In a second main step 310 selected channels are identified in that a plurality of channels are iterated and that target channels are set as selected channels in a fourth sub-step 312. Unlike the first main step 302 this second main step 310 can be made in sequence. Also non-target channels being placed close to target channels are set as selected channels. This may be made by that for each of the non-target channels it is determined if it is placed within a spatial threshold from at least one of the target data channels.

In a third main step 314 an aggregated sensor data package can be formed and transmitted from the neuroprobe. In a fifth sub-step 316 a data package comprising channel sensor data can be formed for the selected channels, in a sixth sub-step 318 the data packages are combined into the aggregated sensor data package, and in a seventh sub-step 320 the aggregated sensor data package is transmitted to a data processing device configured for analyzing the channel sensor data.

Fig 4 generally illustrates a system 400 for analyzing neural activity. The system comprises a neuroprobe 402 and an external data processing device 404 configured for analyzing channel sensor data.

The neuroprobe 402 comprises a plurality of channels 406, such as electrodes, that are configured for capturing sensor data.

In order to identify target channels and non-target channels, that is, channels comprising target data and channels not comprising target data, a target channels identifier 408 can be used. The target channels identifier 408 can be configured for analyzing channel sensor data from the plurality of channels 406 in parallel. If a channel is identified to comprise target data the channel can be assigned as a target channel.

After the channel sensor data has been analyzed by the target channels identifier 408 the different channels can be iterated in a selected channels identifier 410 and each of the plurality of channels assigned as a target channel can be set as a selected channel. Non-target channels spatially close to target channels are also be set as selected channels.

A data transmitter 412 can be provided for forming an aggregated sensor data package by combining channel sensor data package from the selected channels and thereafter transmitting this to the external data processing device 404.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A method for processing a sample set of sensor data in a neuroprobe comprising a plurality of channels, wherein the sample set of sensor data comprises channel sensor data from each of the plurality of channels for a given time period, wherein the plurality of channels comprises target channels, each comprising target data comprising pre-selected features for identifying a spike, and non-target channels, each being without target data, wherein the target channels and the non-target channels are sample specific, said method comprising

   identifying the target channels and the non-target channels by

      for each of the plurality of channels,

         capturing the channel sensor data, determining if the channel sensor data comprises the target data by comparing the channel sensor data with prior channel sensor data, and assigning as target channel if the channel sensor data

      comprises the target data,

   **characterized by**
   identifying selected channels by

      for each of the plurality of channels selecting as selected channel if assigned as target channel, forming and transmitting an aggregated sensor data package by for each of the non-target channels selecting as selected channels if placed within a spatial threshold from at least one of the target data channels, for each of the selected channels forming a data package comprising channel sensor data, combining data packages from the selected channels into the aggregated sensor data package, and transmitting the aggregated sensor data package from the neuroprobe to a data processing device configured for analyzing channel sensor data.

2. The method according to any one of the preceding claims, wherein the channels of the neuroprobe comprise electrodes.

3. The method according to any one of the preceding claims, wherein the target data comprises a spike.

4. The method according to claim 3, wherein the spike is determined by comparing the channel sensor data with a running threshold being a combination of a channel sensor data mean value and a channel sensor data standard deviation value.

5. The method according to any one of the preceding claims, wherein

   the step of identifying the target channels and the non-target channels is made in parallel for the plurality of channels,

the step of identifying selected channels is made in sequence for the plurality of channels, and

the step of forming and transmitting an aggregated sensor data package is made in parallel for the plurality of channels.

6. The method according to any one of the preceding claims, wherein the step of selecting as selected channel if assigned as target channel comprises setting a channel associated bit in a data memory.

7. The method according to any one of the preceding claims, wherein the plurality of channels of the neuroprobe is at least 1000 channels.

8. The method according to any one of the preceding claims,

   wherein the plurality of channels each comprises a unique identifier, and the plurality of channels are divided into sub-groups based on the unique identifiers,

   wherein the step of identifying the target channels and the non-target channels further comprises

   identifying a sub-group, and
   assigning as target channel if another channel within the sub-group is assigned as target channel.

9. A neuroprobe comprising

   a plurality of channels, wherein the plurality of channels comprises target channels, each comprising target data comprising pre-selected features for identifying a spike, and non-target channels, each being without target data, wherein the target channels and the non-target channels are sample specific,

   a target channels identifier for identifying the target channels and the non-target channels by for each of the plurality of channels, capturing a sample set of sensor data, wherein the sample set comprises channel sensor data from each of the plurality of channels for a given time period, determining if the channel sensor data comprises the target data by comparing the channel sensor data with prior channel sensor data, determining if the channel sensor data comprises the target data, and assigning as target channel if the channel sensor data comprises the target data,

   **characterized by**

   a selected channels identifier for identifying selected channels by for each of the plurality of channels selecting as selected channel if assigned as target channel,

   for each of the non-target channels selecting as selected channels if placed within a spatial threshold from at least one of the target data channels,

   and

   a data transmitter for forming and transmitting an aggregated sensor data package by for each of the selected channels forming a data package comprising channel sensor data, combining data packages from the selected channels into the aggregated sensor data package, and transmitting the aggregated sensor data package from the neuroprobe to a data processing device configured for analyzing channel sensor data.

10. The neuroprobe according to claim 9, wherein the channels of the neuroprobe comprise electrodes.

11. The neuroprobe according to any one of the claims 9 to 10, wherein the target data comprises a spike.

12. The neuroprobe according to claim 11, wherein the spike is determined by comparing the channel sensor data with a running threshold being a combination of a channel sensor data mean value and a channel sensor data standard deviation value.

13. The neuroprobe according to any one of the claims 9 to 12, wherein

    the target channels identifier is handling the plurality of channels in parallel,
    the selected channels identifier is handling the plurality of channels in sequence, and
    the data transmitter is handling the plurality of channels in parallel.

14. The neuroprobe according to any one of the claims 9 to 13,
    wherein the step of selecting as selected channel if assigned as target channel comprises setting a channel associated bit in a data memory.

15. The neuroprobe according to any one of the claims 9 to 14,
    wherein the plurality of channels of the neuroprobe is at least 1000 channels.

16. The neuroprobe according to any one of the claims 9 to 15,
    wherein the plurality of channels each comprises a unique identifier, and the plurality of channels are divided into sub-groups based on the unique identifiers, wherein the step of identifying the target channels and the non-target channels further comprises identifying a sub-group, and assigning as target channel if another channel within the sub-group is assigned as target channel.

17. A system for analyzing neural activity, said system comprising

a neuroprobe according to any one of the claims 9 to 16, and
an external data processing device configured for analyzing channel sensor data.

18. The system according to claim 17, wherein the external data processing device is configured for analyzing the channel sensor data by extracting features from the channel sensor data and clustering the features.


**Patentansprüche**

1. Verfahren zum Verarbeiten eines Probensatzes von Sensordaten in einer Nervensonde, die eine Vielzahl von Kanälen umfasst, wobei der Probensatz von Sensordaten Kanalsensordaten von jedem der Vielzahl von Kanälen für einen gegebenen Zeitraum umfasst, wobei die Vielzahl von Kanälen Zielkanäle, die jeweils Zieldaten umfassen, die im Voraus ausgewählte Merkmale zum Identifizieren einer Spitze umfassen, und Nicht-Zielkanäle, die jeweils ohne Zieldaten sind, umfasst, wobei die Zielkanäle und die Nicht-Zielkanäle probenspezifisch sind, wobei das Verfahren umfasst

Identifizieren der Zielkanäle und der Nicht-Zielkanäle durch
für jeden der Vielzahl von Kanälen

Erfassen der Kanalsensordaten,
Bestimmen, ob die Kanalsensordaten die Zieldaten umfassen, durch Vergleichen der Kanalsensordaten mit früheren Kanalsensordaten, und
Zuweisen als Zielkanal, falls die Kanalsensordaten die Zieldaten umfassen,

**gekennzeichnet durch**
Identifizieren ausgewählter Kanäle durch
für jeden der Vielzahl von Kanälen
Auswählen als ausgewählten Kanal, falls dieser als Zielkanal zugewiesen ist,
Bilden und Übertragen eines zusammengefassten Sensordatenpakets durch

für jeden der Nicht-Zielkanäle,
Auswählen als ausgewählte Kanäle, falls sie innerhalb einer räumlichen Schwelle von mindestens einem der Zieldatenkanäle liegen,

für jeden der ausgewählten Kanäle
Bilden eines Datenpakets, das Kanalsensordaten umfasst, Kombinieren von Datenpaketen aus den ausgewählten Kanälen in das zusammengefasste Sensordatenpaket, und Übertragen des zusammengefassten Sensordatenpakets von der Nervensonde an eine Datenverarbeitungsvorrichtung, die zum Analysieren von Kanalsensordaten konfiguriert ist.

2. Verfahren nach einem der vorhergehenden Ansprüche, wobei die Kanäle der Nervensonde Elektroden umfassen.

3. Verfahren nach einem der vorhergehenden Ansprüche, wobei die Zieldaten einen Spike umfassen.

4. Verfahren nach Anspruch 3, wobei der Spike durch Vergleichen der Kanalsensordaten mit einer laufenden Schwelle, die eine Kombination aus einem Mittelwert von Kanalsensordaten und einem Standardabweichungswert von Kanalsensordaten ist, bestimmt wird.

5. Verfahren nach einem der vorhergehenden Ansprüche, wobei

der Schritt des Identifizierens der Zielkanäle und der Nicht-Zielkanäle für die Vielzahl von Kanälen parallel erfolgt,
der Schritt des Identifizierens von ausgewählten Kanälen der Reihe nach für die Vielzahl von Kanälen erfolgt,
der Schritt des Bildens und des Übertragens eines zusammengefassten Sensordatenpakets für die Vielzahl von Kanälen parallel erfolgt.

6. Verfahren nach einem der vorhergehenden Ansprüche, wobei der Schritt des Auswählens als ausgewählter Kanal, falls dieser als Zielkanal zugewiesen wird, das Setzen eines dem Kanal zugeordneten Bits in einem Datenspeicher umfasst.

7. Verfahren nach einem der vorhergehenden Ansprüche, wobei die Vielzahl von Kanälen der Nervensonde mindestens 1000 Kanäle beträgt.

8. Verfahren nach einem der vorhergehenden Ansprüche,

wobei die Vielzahl von Kanälen jeweils eine einzigartige Kennung umfasst, und die Vielzahl von Kanälen basierend auf den einzigartigen Kennungen in Untergruppen unterteilt werden,
wobei der Schritt des Identifizierens der Zielkanäle und der Nicht-Zielkanäle ferner

das Identifizieren einer Untergruppe und das Zuweisen als Zielkanal, falls ein anderer Kanal innerhalb der Untergruppe als Zielkanal zugewiesen wird, umfasst.

9. Nervensonde, umfassend

eine Vielzahl von Kanälen, wobei die Vielzahl von Kanälen Zielkanäle, die jeweils Zieldaten umfassen, die im Voraus ausgewählte Merkmale zum Identifizieren eines Spike umfassen, und Nicht-Zielkanäle, die jeweils keine Zieldaten aufweisen, umfasst, wobei die Zielkanäle und die Nicht-Zielkanäle probenspezifisch sind, eine Zielkanalkennung zum Identifizieren der Zielkanäle und der Nicht-Zielkanäle durch, für jeden der Vielzahl von Kanälen, Erfassen eines Probensatzes von Sensordaten, wobei der Probensatz Kanalsensordaten von jedem der Vielzahl von Kanälen für einen gegebenen Zeitraum umfasst, Bestimmen, ob die Kanalsensordaten die Zieldaten umfassen, durch Vergleichen der Kanalsensordaten mit früheren Kanalsensordaten, Bestimmen, ob die Kanalsensordaten die Zieldaten umfassen, und Zuweisen als Zielkanal, falls die Kanalsensordaten die Zieldaten umfassen,
**gekennzeichnet durch**
eine ausgewählte Kanalkennung zum Identifizieren ausgewählter Kanäle durch, für jeden der Vielzahl von Kanälen, Auswählen als ausgewählten Kanal, falls dieser als Zielkanal zugewiesen ist,
für jeden der Nicht-Zielkanäle
Auswählen als ausgewählte Kanäle, falls sie innerhalb einer räumlichen Schwelle von mindestens einem der Zieldatenkanäle angeordnet sind, und
einen Datensender zum Bilden und Übertragen eines zusammengefassten Sensordatenpakets durch, für jeden der ausgewählten Kanäle, Bilden eines Datenpakets, das Kanalsensordaten umfasst, Kombinieren von Datenpaketen aus den ausgewählten Kanälen in das zusammengefasste Sensordatenpaket, und Übertragen des zusammengefassten Sensordatenpakets von der Nervensonde an eine Datenverarbeitungsvorrichtung, die zum Analysieren von Kanalsensordaten konfiguriert ist.

10. Nervensonde nach Anspruch 9, wobei die Kanäle der Nervensonde Elektroden umfassen.

11. Nervensonde nach einem der Ansprüche 9 bis 10, wobei die Zieldaten einen Spike umfassen.

12. Nervensonde nach Anspruch 11, wobei der Spike durch Vergleichen der Kanalsensordaten mit einer laufenden Schwelle, die eine Kombination aus einem Mittelwert von Kanalsensordaten und einem Standardabweichungswert der Kanalsensordaten ist, bestimmt wird.

13. Nervensonde nach einem der Ansprüche 9 bis 12, wobei

die Zielkanalkennung die Vielzahl von Kanälen parallel handhabt,
die ausgewählte Kanalkennung die Vielzahl von Kanälen nacheinander handhabt, und
der Datensender die Vielzahl von Kanälen parallel handhabt.

14. Nervensonde nach einem der Ansprüche 9 bis 13, wobei der Schritt des Auswählens als ausgewählten Kanal, falls dieser als Zielkanal zugewiesen ist, das Setzen eines dem Kanal zugeordneten Bits in einem Datenspeicher umfasst.

15. Nervensonde nach einem der Ansprüche 9 bis 14, wobei die Vielzahl von Kanälen der Nervensonde mindestens 1000 Kanäle beträgt.

16. Nervensonde nach einem der Ansprüche 9 bis 15, wobei die Vielzahl von Kanälen jeweils eine einzigartige Kennung umfasst und die Vielzahl von Kanälen basierend auf den einzigartigen Kennungen in Untergruppen unterteilt ist, wobei der Schritt des Identifizierens der Zielkanäle und der Nicht-Zielkanäle ferner das Identifizieren einer Untergruppe und das Zuweisen als Zielkanal umfasst, falls ein anderer Kanal innerhalb der Untergruppe als Zielkanal zugewiesen wird.

17. System zum Analysieren einer Nervenaktivität, wobei das System umfasst

eine Nervensonde nach einem der Ansprüche 9 bis 16 und
eine externe Datenverarbeitungsvorrichtung, die zum Analysieren von Kanalsensordaten konfiguriert ist.

18. System nach Anspruch 17, wobei die externe Datenverarbeitungsvorrichtung zum Analysieren der Kanalsensordaten durch Entnehmen von Merkmalen aus den Kanalsensordaten und Gruppieren der Merkmale konfiguriert ist.

**Revendications**

1. Procédé de traitement d'un ensemble d'échantillons de données de capteur dans une neurosonde comprenant une pluralité de canaux, dans lequel l'ensemble d'échantillons de données de capteur comprend des données de capteur de canal provenant de chacun de la pluralité de canaux pour une période de temps donnée, dans lequel la pluralité de canaux comprend des canaux cibles, chacun comprenant des données cibles comprenant des caractéristiques présélectionnées pour identifier un pic, et des canaux non cibles, chacun étant dépourvu de données cibles,das lequel les canaux cibles et les canaux non cibles sont spécifiques à un échantillon, ledit procédé comprenant de :

identifier les canaux cibles et les canaux non cibles en

pour chacun de la pluralité de canaux,
capturant les données du capteur de canal,
déterminant si les données du capteur de canal comprennent les données cibles en comparant les données du capteur de canal avec les données de capteur de canal antérieures, et
attribuant en tant que canal cible si les données du capteur de canal comprennent les données cibles,

**caractérisé par** l'identification des canaux sélectionnés en

pour chacun de la pluralité de canaux,
sélectionnant comme canal sélectionné s'il est attribué comme canal cible,
formant et transmettant un ensemble de données de capteur agrégées en pour chacun des canaux non cibles,
sélectionnant comme canaux sélectionnés s'il est placé à l'intérieur d'un seuil spatial à partir d'au moins un des canaux de données cibles,
pour chacun des canaux sélectionnés,
formant un paquet de données comprenant des données de capteur de canal, combinant des paquets de données provenant des canaux sélectionnés en le paquet de données de capteur agrégées, et transmettant le paquet de données de capteur agrégées de la neurosonde au dispositif configuré pour analyser les données du capteur de canal.

2. Procédé selon une quelconque des revendications précédentes, dans lequel les canaux de la neurosonde comprennent des électrodes.

3. Procédé selon une quelconque des revendications précédentes, dans lequel les données cibles comprennent un pic.

4. Procédé selon la revendication 3, dans lequel le pic est déterminé en comparant les données du capteur de canal avec un seuil courant qui est une combinaison d'une valeur moyenne des données du capteur de canal et d'une valeur d'écart type des données du capteur de canal.

5. Procédé selon une quelconque des revendications précédentes, dans lequel l'étape d'identification des canaux cibles et des canaux non cibles est réalisée en parallèle pour la pluralité de canaux,

l'étape d'identification des canaux sélectionnés est effectuée en séquence pour la pluralité de canaux, et
l'étape de formation et de transmission d'un paquet de données de capteur agrégées est effectuée en parallèle pour la pluralité de canaux.

6. Procédé selon une quelconque des revendications précédentes, dans lequel l'étape de sélection comme canal sélectionné s'il est attribué comme canal cible comprend la définition d'un bit associé au canal dans une mémoire de données.

7. Procédé selon une quelconque des revendications précédentes, dans lequel la pluralité de canaux de la neurosonde est d'au moins 1000 canaux.

8. Procédé selon une quelconque des revendications précédentes, dans lequel la pluralité de canaux comprend chacun un identifiant unique et la pluralité de canaux est divisée en sous-groupes sur la base des identifiants uniques,
dans lequel l'étape d'identification des canaux cibles et des canaux non cibles comprend en outre l'identification d'un sous-groupe, et l'attribution comme canal cible si un autre canal du sous-groupe est attribué comme canal cible.

9. Neurosonde comprenant

une pluralité de canaux, dans laquelle la pluralité de canaux comprend des canaux cibles, chacun comprenant des données cibles comprenant des caractéristiques présélectionnées pour identifier un pic, et des canaux non cibles, chacun étant sans données cibles, dans laquelle les canaux cibles et les canaux non cibles sont spécifiques à l'échantillon,
un identifiant de canaux cibles pour identifier les canaux cibles et les canaux non cibles en, pour chacun de la pluralité de canaux, capturant un ensemble d'échantillons de données de capteur, dans lequel l'ensemble d'échantillons comprend des données de capteur de canal provenant de chacun de la pluralité de canaux pour une période de temps donnée, déterminant si les données du capteur de canal comprennent les données cibles en comparant les données de capteur de canal avec les données de capteur de canal antérieures, déterminant si les données de capteur de canal comprennent les données cibles, et attribuant comme canal cible si les données du capteur de canal comprennent les données cibles,
**caractérisé par**
un identifiant de canaux sélectionnés pour identifier les canaux sélectionnés en sélectionnant pour chacun de la pluralité de canaux comme canal sélectionné s'il est attribué comme canal cible,
pour chacun des canaux non cibles
en sélectionnant comme canaux sélectionnés s'il est placé à l'intérieur d'un seuil spatial d'au moins un des canaux de données cibles et
un émetteur de données pour former et transmettre un paquet de données de capteur agrégées en formant, pour chacun des canaux sélectionnés, un paquet de données comprenant des données de capteur de canal, en combinant des paquets de données provenant des canaux sélectionnés dans le paquet de données de capteur agrégées, et transmettant le paquet de données de capteur agrégées de la neurosonde à un dispositif configuré pour analyser les données de capteur de canal.

10. Neurosonde selon la revendication 9, dans laquelle les canaux de la neurosonde c comprennent des électrodes.

11. Neurosonde selon une quelconque des revendications 9 à 10, dans laquelle les données cibles comprennent un pic.

12. Neurosonde selon la revendication 11, dans laquelle le pic est déterminé en comparant les données du capteur de canal avec un seuil de courant qui est une combinaison d'une valeur moyenne de données de capteur de canal et d'une valeur d'écart type de données de capteur de canal.

13. Neurosonde selon une quelconque des revendications 9 à 12, dans laquelle
l'identifiant des canaux cibles gère la pluralité de canaux en parallèle, l'identifiant de canaux sélectionné gère la pluralité de canaux en séquence, et l'émetteur de données gère la pluralité de canaux en parallèle.

14. Neurosonde selon une quelconque des revendications 9 à 13, dans laquelle l'étape de sélection comme canal sélectionné s'il est attribué comme canal cible comprend la définition d'un bit associé au canal dans une mémoire de données.

15. Neurosonde selon une quelconque des revendications 9 à 14, dans laquelle la pluralité de canaux de la neurosonde est d'au moins 1000 canaux.

16. Neurosonde selon une quelconque des revendications 9 à 15, dans laquelle la pluralité de canaux comprend chacun un identifiant unique, et la pluralité de canaux est divisée en sous-groupes sur la base des identifiants uniques, dans lequel l'étape d'identification des canaux cibles et des canaux non cibles comprend en outre l'identification d'un sous-groupe et son attribution en tant que canal cible si un autre canal du sous-groupe est attribué comme canal cible.

17. Système d'analyse de l'activité neuronale, ledit système comprenant :

une neurosonde selon une quelconque des revendications 9 à 16 et
un dispositif de traitement de données externe configuré pour analyser les données de capteur de canal.

18. Système selon la revendication 17, dans lequel le dispositif de traitement de données externes est configuré pour analyser les données du capteur de canal en extrayant des caractéristiques des données du capteur de canal et en regroupant les caractéristiques.

Fig 1

Fig 2

300 —

Identifying target channels ～302

Capturing channel sensor data ～304

Determining if channel sensor data comprises target data ～306

Assigning as target channel if comprising target data ～308

Identifying selected channels ～310

Selecting as selected channel if target channel ～312

Forming and transmitting aggregated sensor data package ～314

Forming data package comprising channel sensor data ～316

Combining data packages from selected channels ～318

*Fig 3*

Transmitting aggregated sensor data package ～320

400 ～

404

*Fig 4*

412 ～402

410

408

406

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- A low-power integrated neural interface with digital spike detection and extraction. **BENOIT GOSSELIN et al.** ANALOG INTEGRATED CIRCUITS AND ISGNAL PROCESSING. KLUWER ACADEMIC PUBLISHERS, 15 August 2009, vol. 64, 3-11

- Automatic channel selection in neural microprobes: A combinatorial multi-armed bandit approach. **GORDILLO CAMILO et al.** 2016 IEEE/RSJ INTERNATIONAL CONFERENCE ON INTELLIGENT ROBOTS AND SYSTEMS (IROS). IEEE, 09 October 2016, 1844-1850